# FASCICULE DE BREVET EUROPEEN

(11) **EP 2 897 692 B1**
(45) Date de publication et mention de la délivrance du brevet: **14.06.2017**
(21) Numéro de dépôt: 13773303.6
(22) Date de dépôt: 16.09.2013
(51) Int. Cl.: A61Q 5/00, A61Q 5/12, A61K 8/06, A61K 8/25, A61K 8/26, A61K 8/34, A61K 8/92

(54) **COMPOSITION COSMÉTIQUE SOUS FORME D'ÉMULSION ET PROCÉDÉ DE TRAITEMENT COSMÉTIQUE**
KOSMETISCHE ZUSAMMENSETZUNG IN EMULSIONSFORM UND KOSMETISCHES BEHANDLUNGSVERFAHREN
COSMETIC COMPOSITION IN EMULSION FORM AND COSMETIC TREATMENT PROCESS

(30) Priorité: 21.09.2012 FR 1258877; 16.11.2012 US 201261727144 P
(43) Date de publication de la demande: 29.07.2015
(73) Titulaire: L'Oréal, 75008 Paris (FR)
(72) Inventeur: DEGOUL, Marie-Cécile, F-75003 Paris (FR); PROUST, Cécile, F-75015 Paris (FR); BARA, Isabelle, F-94210 La Varenne St Hilaire (FR)
(74) Mandataire: Dodin, Catherine
(86) Numéro de dépôt international: PCT/FR2013/052115
(87) Numéro de publication internationale: WO 2014/044957

(56) Documents cités:
- EP-A1- 1 477 159
- EP-A1- 2 149 361
- EP-A2- 1 310 235
- WO-A1-98/07404
- WO-A1-98/42300
- WO-A1-98/42301
- WO-A1-02/096378
- FR-A1- 2 208 642

## Description

La présente invention concerne des compositions cosmétiques notamment capillaires, pouvant se présenter sous forme de perles en suspension dans un liquide s'utilisant de préférence après agitation.

Les compositions de soin capillaire permettent généralement d'apporter de la cosmétique au cheveu, tel que du démêlage, de la douceur ou un toucher lisse, par exemple.
Toutefois, les compositions de soin capillaire peuvent, dans certains cas, conférer de l'alourdissement aux cheveux, notamment lorsque les formulations contiennent une forte concentration d'huile, généralement associée à un apport de brillance. En outre, ces compositions, bien que généralement parfumées afin de rendre l'application agréable, ne permettent pas toutefois un parfumage des cheveux intense et rémanent.
De plus, ces compositions se présentent le plus souvent sous forme d'émulsion crème ou de liquide monophasique, et il n'est pas aisé de disposer d'autres formes galéniques.

La présente invention a pour but de pallier les inconvénients de l'art antérieur et donc de proposer des compositions cosmétiques susceptibles d'apporter de la cosmétique aux matières kératiniques, telles que les cheveux, notamment de la brillance et du soin, ainsi qu'un parfumage adéquat desdites matières kératiniques, tout en n'apportant pas d'alourdissement des cheveux, ces compositions ayant par ailleurs une galénique diversifiée et visuellement innovante et attractive.

La présente invention a donc pour objet une composition cosmétique sous forme d'émulsion huile-dans-eau, comprenant :
- une phase hydroalcoolique continue comprenant de l'eau et au moins un monoalcool en C₁-C₄; l'eau étant présente dans la composition à une teneur allant 0,5 à 10% en poids par rapport au poids total de la composition;
- de 0,1 à 10% en poids, par rapport au poids total de la composition, d'une ou plusieurs huiles végétales de type triglycérides; et
- des particules choisies parmi les particules de silicate insoluble, les particules de silice, et leurs mélanges; lesdites particules présentant une taille inférieure ou égale à 10 µm.

Les compositions selon la présente invention reposent sur le principe des émulsions de Pickering.
Il est connu par la demande FR2208642 des compositions cosmétiques liquides à deux phases, dites de type Emulsion « Pickering », attrayantes au regard du fait que la phase huileuse est dispersée sous la forme de sphères (ou billes, ou perles) dans un mélange homogène hydroalcoolique. Des particules solides finement divisées sont adsorbées dans l'interface entre l'huile et le mélange homogène hydroalcoolique permettant de stabiliser les sphères huileuses.
Au sens de la présente invention, une émulsion de Pickering est une dispersion de deux phases non miscibles entre elles, ne comprenant pas de tensioactif, et stabilisée par des particules colloïdales.
Dans le cadre de la présente invention, les deux phases non miscibles sont, d'une part une phase hydroalcoolique, et d'autre part une phase huileuse; les particules stabilisantes étant des silices ou des silicates.

Les compositions selon l'invention sont très intéressantes et innovantes en terme de parfumage des cheveux, d'apport de brillance, de soin et de conditionnement. Elles possèdent un aspect visuel nouveau (composition biphase, coloré ou non, dont une des phases est formée ou comprend des billes); l'aspect de perles en suspension est particulièrement attrayant, notamment par rapport aux compositions traditionnelles de soin ou conditionnement capillaires.
Les compositions selon l'invention possèdent en outre l'avantage d'éviter l'emploi de tensioactifs; l'absence de ces composés permet de s'affranchir d'un certain nombre d'inconvénients, tels que ceux énoncés dans la demande FR2208642. Les compositions selon l'invention peuvent en outre être aisément préparées à froid ou à température ambiante contrairement aux techniques connues de fabrication d'émulsions généralement préparées à chaud. Les compositions de l'invention peuvent ainsi être obtenues par simple agitation, par exemple à l'aide d'un mélangeur à pales.

En résumé, les compositions selon l'invention permettent d'obtenir des galéniques au visuel très intéressant et diversifié. Il est possible d'obtenir des formules biphasées comportant une phase continue limpide colorée ou non, et une phase dispersée sous forme de billes ou de "perles" dont la taille et la position peuvent varier en fonction de la nature et des quantités des matières premières la composant. On peut ainsi obtenir des performances sur cheveux comme un apport intense de brillance et de soin. Il est aussi possible d'introduire une forte quantité de parfum permettant en même temps le parfumage des cheveux.
La composition sous forme d'émulsion ainsi obtenue est très stable thermiquement et dans le temps; lors de l'utilisation de la composition, on peut secouer l'émulsion qui est capable de s'homogénéiser très facilement, puis de reprendre en quelques minutes, son aspect initial sous forme de perles.

De manière préférentielle, la composition selon l'invention se présente sous forme liquide. Au sens de la présente invention, on entend par "composition liquide", une composition ne se présentant pas sous une forme solide, dont la viscosité mesurée à l'aide d'un viscosimètre Rhéomat 180 à 25°C à la vitesse de rotation de 200 tours/min après 10 minutes de rotation est de préférence inférieure ou égale à 2 Pa.s, préférentiellement comprise entre 0,01 et 0,5 Pa.s.
De manière préférentielle, la composition selon l'invention ne comprend pas (0%) de tensioactif. Au sens de la présente invention, on entend par "tensioactif", une molécule amphiphile c'est-à-dire présentant deux parties de polarité différente, l'une lipophile (qui retient les matières grasses) et apolaire, l'autre hydrophile (miscible dans l'eau) et polaire. Les particules solides employées dans le cadre de l'invention ne sont pas considérées comme des tensioactifs au sens de la présente invention.
Ainsi qu'indiqué ci-dessus, la composition selon l'invention se présente sous forme d'une émulsion huile-dans-eau, qui comprend une phase hydroalcoolique continue et une phase huileuse dispersée.

### Phase huileuse dispersée

La composition selon l'invention comprend une ou plusieurs huiles végétales de type triglycérides. La/les huile(s) végétale(s) de type triglycérides sont présentes dans la composition à une concentration limitée, afin de limiter l'effet d'alourdissement et de toucher gras de la chevelure susceptible d'être lié à leur présence. Ainsi, la composition selon l'invention comprend de 0,1 à 10% en poids, par rapport au poids total de la composition, d'une ou plusieurs huiles végétales de type triglycérides; mieux de 0,2 à 8% en poids, notamment de 0,5 à 6% en poids.

Il est bien connu que les huiles végétales comprennent principalement des triglycérides, qui sont des glycérides dans lesquels les trois groupements hydroxyle du glycérol sont estérifiés par des acides gras, généralement en C8-C22.
De préférence, les huiles végétales susceptibles d'être employées dans le cadre de la présente invention sont choisies parmi les huiles de type triglycérides dont la teneur en acide gras en C16:0 (acide palmitique) est supérieure ou égale à 7,5% en poids par rapport au poids total d'acides gras desdits triglycérides.
On peut notamment citer l'huile d'olive; l'huile d'avocat; l'huile d'argan; l'huile de camélia, l'huile de graines de coton, l'huile de son de riz, l'huile de germes de maïs, l'huile de germes de blé, l'huile de passiflore, l'huile de soja, l'huile d'arachide, l'huile de coprah, l'huile de graine de citrouille, l'huile de sésame, l'huile de babassu, l'huile de coco, ainsi que leurs mélanges.
On peut bien évidemment également utiliser, seules ou en mélange avec les huiles ci-dessus, des huiles végétales de type triglycérides dont la teneur en acide gras en C16:0 (acide palmitique) est inférieure à 7,5% en poids par rapport au poids total d'acides gras desdits triglycérides.
On peut ainsi citer l'huile de colza, l'huile d'amande, l'huile de lin, l'huile de ricin, l'huile de carthame et l'huile de graine de tournesol, ainsi que leurs mélanges.

On peut encore citer l'huile de palme qui est généralement sous forme pâteuse/solide, et qui peut être utilisée de préférence en mélange avec une autre huile, notamment végétale, et de préférence liquide.
On peut également employer un mélange de ces différentes huiles végétales. Préférentiellement, l'huile végétale peut être choisie parmi l'huile d'argan, l'huile d'avocat et l'huile de camélia.

Dans un mode de réalisation préféré, la composition comprend au moins une huile végétale de type triglycérides dont la teneur en acide gras en C16:0 (acide palmitique) est supérieure ou égale à 7,5%; et tout particulièrement de l'huile d'avocat, de l'huile d'argan et/ou de l'huile de camélia.

La composition peut également comprendre une ou plusieurs huiles additionnelles choisies parmi les huiles hydrocarbonées apolaires, dans la mesure où celles-ci n'altèrent pas la stabilité de la composition selon l'invention.
Par "huile apolaire" au sens de la présente invention, on entend une huile dont le paramètre de solubilité à 25°C δa est égal à 0 (J/cm3)½.
La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).
Selon cet espace de Hansen :
- δD caractérise les forces de dispersion de LONDON issues de la formation de dipôles induits lors des chocs moléculaires ;
- δp caractérise les forces d'interactions de DEBYE entre dipôles permanents ainsi que les forces d'interactions de KEESOM entre dipôles induits et dipôles permanents;
- δh caractérise les forces d'interactions spécifiques (type liaisons hydrogène, acide/base, donneur/accepteur, etc.) ; et
- δa est déterminé par l'équation : δa = (δp² + δh²)½.
Les paramètres δp, δh, δD et δa sont exprimés en (J/cm3)½.
Par "huile hydrocarbonée", on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide. Selon un mode de réalisation, l'huile hydrocarbonée apolaire selon l'invention est dépourvue d'hétéroatome(s). On entend par hétéroatome, un atome différent du carbone ou de l'hydrogène.
Parmi les huiles hydrocarbonées apolaires, on peut citer les alcanes saturés (ou hydrocarbures), linéaires ou ramifiés, et en particulier les hydrocarbures linéaires ou ramifiés d'origine minérale ou synthétique, de préférence une huile de paraffine liquide, volatile ou non volatile; les isoparaffines hydrogénées ou non; l'huile de naphtalène, un polydécène liquide totalement ou partiellement hydrogéné, l'isoeicosane, un copolymère décène/butène, un polyisobutène hydrogéné, ou un copolymère poly-butène/poly-isobutène et leurs mélanges.
On peut tout particulièrement citer les huiles hydrocarbonées apolaires suivantes :
- les isoparaffines hydrogénées, tel que par exemple le polyisobutène hydrogéné, vendu sous la dénomination de Parléam par la société Rossow,
- l'isoparaffine C₈-C₉ vendue sous la dénomination de Isopar E par Exxon Mobil Chemical ; l'isoparaffine C₁₁-C₁₃ de EXXON vendue sous la dénomination de l'Isopar L, ou l'isoparaffine C₁₃-C₁₄ vendue sous le nom d'Isopar M par la société Exxon Mobil Chemical,
- les paraffines liquides de Petro Canada : Puretol 9, ou le Blandol de Sonneborn, ou le Marcol 82 commercialisé par Exxon Mobil Chemical, et
- les perhydrosqualènes végétaux vendus sous la dénomination d'olive squalane par SOS corporation Alimentaria, ou Vegetable squalane par lake oil, ou Exolive par Caroi Line Cosmetica.
De préférence, on utilise les isoparaffines hydrogénées ou non.

La composition peut également comprendre une ou plusieurs huiles additionnelles polaires notamment choisies parmi les alcools gras liquides et notamment les alcools gras insaturés comme l'alcool oléique et les alcools gras ramifiés comme l'alcool isostéarylique ou l'octyldodécanol.
Enfin on peut citer comme huiles additionnelles susceptibles d'être employées, les esters liquides autres que les triglycérides comme le myristate d'isopropyle.

La ou les huiles additionnelles peuvent être présentes dans la composition selon l'invention à une teneur allant de 0,1 à 70% en poids, notamment de 1 à 50% en poids, par rapport au poids total de la composition (lorsqu'elles sont présentes).

La composition selon l'invention peut également comprendre, notamment dans la phase huileuse dispersée, au moins un colorant liposoluble, qui peut être d'origine naturelle ou synthétique.
Par "colorant liposoluble", on entend selon l'invention, tout composé généralement organique, naturel ou synthétique, soluble à au moins 0,1% en poids, à 25°C, dans la phase huileuse.
On peut par exemple citer :
- un colorant organique violet, le D&C VIOLET N° 2 K7014 (nom chimique : Pourpre d'alizurol SS) ;
- un colorant organique vert, le D&C GREEN N°6 K7016 / 90097 D&C GREEN 6. (nom chimique : vert de quinizarin E SS) ;
- un colorant organique rose, le D&C RED N°21 K7061 / SUNCROMA D&C RED 21 C 14-032 (nom chimique : Eosine); et
- un colorant végétal orange, la BETATENE 30% OLV (nom chimique : Carote-noides (CI.75130-E160 A) à 30% dans l'huile d'olive.
Les colorants liposolubles conformes à l'invention peuvent être également choisis parmi le rouge Soudan, le D&C Red 17, le β-carotène, le brun Soudan, le D&C Yellow 11, le D&C Orange 5, le jaune quinoléine et le rocou.

La phase huileuse dispersée selon l'invention se présente de préférence sous la forme de sphères de taille plus ou moins homogène, pouvant varier de 0,5 à 20 mm, de préférence allant de 0,5 à 10 mm, préférentiellement de 1 à 5 mm.
Le nombre de sphères huileuses peut varier en fonction de la quantité de phase huileuse et de particules de silice ou silicate présentes. On peut en compter de 1 à plusieurs millions par litre de formulation en fonction de leur taille. Ces sphères peuvent être présentes dans la partie supérieure ou inférieure de la composition les comprenant ou dans les deux parties de la composition.

### Phase hydroalcoolique continue

La composition selon l'invention comprend, en plus d'une phase huileuse dispersée, une phase hydroalcoolique continue comprenant au moins un monoalcool en C₁-C₄.
Par "phase hydroalcoolique", on entend une phase comprenant de l'eau et un ou plusieurs monoalcools en C1-C4.
Comme monoalcool conforme à l'invention, on peut citer de préférence les mono-hydroxyalkyles linéaires en C₁-C₄, et tout particulièrement l'éthanol.
Le ou les monoalcools peuvent être présents dans la composition à une teneur allant de 20 à 80% en poids, de préférence de 40 à 70% en poids, par rapport au poids total de la composition.
L'eau est présente dans la composition à une teneur allant de 0,5 à 10% en poids, préférentiellement de 1 à 5% en poids, par rapport au poids total de la composition selon l'invention.
La composition peut en outre comprendre, dans la phase hydroalcoolique continue, au moins un colorant hydrosoluble, qui peut être d'origine synthétique ou végétale.
Par "colorant hydrosoluble", on entend au sens de l'invention tout composé, généralement organique, naturel ou synthétique, soluble à au moins 0,1% en poids, à 25°C, dans une phase aqueuse ou dans les solvants miscibles à l'eau tels que les monoalcools en C₁-C₄ et apte à colorer. On peut notamment citer les colorants hydrosolubles synthétiques ou naturels tels que par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétanine (betterave), le carmin, la chlorophylline cuivrée, le bleu de méthylène, les anthocyanines (enocianine, carotte noire, hibiscus, sureau), le caramel et la riboflavine.

Selon une forme particulièrement préférée de l'invention, la phase huileuse dispersée et la phase hydroalcoolique continue sont ajustées de telle sorte que la valeur absolue de la différence de densité (Δd) à 25°C, entre la phase huileuse dispersée et la phase hydroalcoolique continue est d'au plus 0,05, avantageusement d'au plus 0,01.

### Particules

La composition selon l'invention comprend également des particules destinées à la stabiliser, en se positionnant à l'interface phase dispersée/phase continue.
Ces particules sont solides et sont choisies parmi les particules de silicate insolubles, les particules de silice, ainsi que leurs mélanges, lesdites particules présentant une taille inférieure ou égale à 10 µm, notamment allant de 1 à 10 µm, préférentiellement de 1,5 à 8 µm.

Les particules utilisées dans la composition cosmétique selon l'invention peuvent être traitées en surface par un agent organique.
Notamment, les particules peuvent avoir subi totalement ou partiellement un traitement de surface de nature chimique, électronique, électrochimique, mécano-chimique ou mécanique, avec un agent organique tel que ceux décrits notamment dans Cosmetics and Toiletries, Février 1990, Vol. 105, p. 53-64.
Ces agents organiques peuvent être par exemple choisis parmi les cires, par exemple la cire de carnauba et la cire d'abeille; les acides gras, les alcools gras et leurs dérivés, tels que l'acide stéarique, l'acide hydroxystéarique, l'alcool stéarylique, l'alcool hydroxystéarylique, l'acide laurique et leurs dérivés; les tensioactifs anioniques; les lécithines; les sels de sodium, potassium, magnésium, fer, titane, zinc ou aluminium d'acides gras, par exemple le stéarate ou laurate d'aluminium; les alcoxydes métalliques; le polyéthylène; les polymères ou copolymères (méth)acryliques, par exemple les polyméthyl(méth)acrylates; les alcanoamines; les composés siliconés tels que les silicones, les polydiméthylsiloxanes; les composés organiques fluorés tels que les perfluoroalkyleéthers; les composés fluorosiliconés.
Les particules traitées en surface peuvent aussi avoir été traitées par un mélange de ces composés et/ou avoir subi plusieurs traitements de surface.
Les particules traitées en surface peuvent être préparés selon des techniques de traitement de surface bien connues de l'homme de l'art ou trouvés tels quels dans le commerce. Le traitement en surface peut ainsi être réalisé par exemple par réaction chimique d'un agent de surface avec la surface des particules et création d'une liaison covalente entre l'agent de surface et les particules. Cette méthode est notamment décrite dans le brevet US 4 578 266.
De préférence, on utilisera un agent organique lié aux particules de manière covalente.
L'agent organique pour le traitement de surface peut représenter de 0,1 à 50% en poids du poids total de la particule traitée en surface, de préférence de 0,5 à 30% en poids, et préférentiellement de 1 à 10% en poids.
De préférence, les traitements en surface des particules sont choisis parmi les traitements suivants :
- un traitement PEG-Silicone comme le traitement de surface AQ commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement copolymère Acrylate comme le traitement de surface APD commercialisé par Daito ;
- un traitement Phosphate de Perfluoroalkyle/Isopropyl Titanium Triisostéarate comme le traitement de surface PF + ITT commercialisé par Daito.

Les particules susceptibles d'être utilisées dans le cadre de l'invention peuvent être des particules de silice. Ainsi, la composition peut comprendre plus particulièrement une ou plusieurs silices hydrophiles ou hydrophobes, ou leurs mélanges.

On entend par "silice hydrophile" aussi bien les silices hydrophiles pures que les particules totalement ou partiellement enrobées de silice hydrophile.
Les silices hydrophiles susceptibles d'être utilisées sont de préférence amorphes. Elles se présentent généralement sous forme pulvérulente. Elles peuvent en outre être d'origine pyrogénée ou d'origine précipitée. Les silices pyrogénées sont obtenues habituellement par pyrolyse en flamme continue à 1000°C du tétrachlorure de silicium (SiCl₄) en présence d'hydrogène et d'oxygène. Les silices précipitées sont obtenues plus particulièrement par réaction d'un acide sur des solutions de silicates alcalins, de préférence du silicate de sodium.

Dans un premier mode de réalisation de l'invention, la silice hydrophile peut être choisie parmi les silices hydrophiles ayant une dimension moyenne de particules en nombre allant de 3 à 50 nm. On peut en particulier citer les silices hydrophiles décrites dans les tableaux ci-dessous, et leurs mélanges.

| Nom commercial | AEROSIL 90 (Société Degussa-Hüls) | AEROSIL 130 (Société Degussa-Hüls) | AEROSIL 150 (Société Degussa-Hüls) | AEROSIL 200 (Société Degussa-Hüls) |
|---|---|---|---|---|
| Mode d'obtention | Pyrogénation | Pyrogénation | Pyrogénation | Pyrogénation |
| Dimension moyenne des particules (nm) | 20 | 16 | 14 | 12 |
| Remarque | | | | taille agrégats : 10-30 µm et 200 µm |

| Nom commercial | AEROSIL 300 (Société Degussa-Hüls) | AEROSIL 380 (Société Degussa-Hüls) | AEROSIL OX 50 (Société Degussa-Hüls) | SILICE FK 320 DS (Société Degussa-Hüls |
|---|---|---|---|---|
| Mode d'obtention | Pyrogénation | Pyrogénation | Pyrogénation | Précipitation |
| Dimension moyenne des particules (nm) | 7 | 7 | 40 | 18 |

La silice hydrophile susceptible d'être utilisée peut également consister en une particule, notamment en une particule minérale, recouverte totalement ou partiellement de silice.
On peut citer en particulier les billes de silice contenant de l'oxyde de titane, par exemple commercialisées sous la dénomination TORAYCERAM S-IT® par la société Toray; la silice enrobée de dioxyde de titane et recouvert de silice poreuse (85/5/10) (taille: 0,6 µm), notamment commercialisée sous la dénomination ACS-0050510® par la société SACI-CFPA; le nano-oxyde de titane anatase traité alumine et silice à 40% dans l'eau (taille: 60 nm, monodisperse), notamment commercialisé sous la dénomination MIRASUN TIW 60® par la société Rhodia Chimie; le nano-oxyde de titane anatase (60 nm) enrobé silice/alumine/cérium IV 15/5/3 en dispersion aqueuse à 32%, notamment commercialisé sous la dénomination MIRASUN TIW 160® par la société Rhodia Chimie; le nano-oxyde de titane anatase traité alumine et silice (34/4,3/1,7) en dispersion aqueuse à 40%, notamment commercialisé sous la dénomination TIOVEIL AQ-N® par la société Uniqema; le nano-oxyde de titane enrobé de silice (66/33) (granulométrie du dioxyde de titane : 30 nm ; épaisseur de silice : 4 nm), notamment commercialisé sous la dénomination MAXLIGHT TS-04® par la société Nichimen Europe PLC; le nano-oxyde de titane enrobé de silice (80/20) (granulométrie dioxyde de titane : 30 nm; épaisseur de silice : 2 nm) notamment commercialisé sous la dénomination MAXLIGHT TS-042® par la société Nichimen Europe PLC.

Dans une variante de l'invention, on peut utiliser comme silice hydrophile, les silices pyrogénées et en particulier celles commercialisées sous les dénominations AEROSIL (nom INCI : silica) et notamment celle commercialisée sous la dénomination AEROSIL 200 par la société Degussa-Hüls.

On entend par "silice hydrophobe", aussi bien les silices hydrophobes pures que les particules totalement ou partiellement enrobées de silice hydrophobe.
Les silices hydrophobes susceptibles d'être utilisées sont de préférence amorphes et d'origine pyrogénée. Elles se présentent de préférence sous forme pulvérulente.

Les silices hydrophobes amorphes d'origine pyrogénée sont généralement obtenues à partir de silices pyrogénées hydrophiles. Ces dernières sont obtenues par pyrolyse en flamme continue à 1000°C du tétrachlorure de silicium (SiCl4) en présence d'hydrogène et d'oxygène. Elles sont ensuite rendues hydrophobes par un traitement avec des silanes halogénés, des alcoxysilanes ou des silazanes. Les silices hydrophobes diffèrent des silices hydrophiles de départ par, entre autre, une densité de groupe silanols plus faible et par une adsorption de vapeur d'eau plus petite.

Dans un second mode de réalisation de l'invention, la silice hydrophobe est choisie parmi les silices ayant une dimension moyenne de particules en nombre allant de 3 à 50 nm. On peut en particulier citer les silices hydrophobes décrites dans le tableau ci-dessous, et leurs mélanges.

| Nom commercial | AEROSIL R202 (Société Degussa-Hüls) | AEROSIL R805 (Société Degussa-Hüls) | AEROSIL R812 (Société Degussa-Hüls) | AEROSIL R972 (Société Degussa-Hüls) | AEROSIL R974 (Société Degussa-Hüls) |
|---|---|---|---|---|---|
| Dimension moyenne des particules (nm) | 14 | 12 | 7 | 16 | 12 |

La silice hydrophobe susceptible d'être utilisée peut consister également en une particule, notamment minérale, telle que les pigments et oxydes métalliques, recouverts totalement ou partiellement de silice hydrophobe.
Dans une variante de l'invention, on peut utiliser comme silice hydrophobe, une silice pyrogénée hydrophobe traitée en surface par un diméthylsiloxane, comme celle commercialisée sous la dénomination AEROSIL R972 (nom INCI : Silica Dimethyl Silylate) par la société Degussa-Hüls.

Dans un troisième mode de réalisation de l'invention, la silice peut être choisie parmi les silices ayant une dimension moyenne de particules en nombre supérieure à 1 µm. On peut ainsi citer les silices proposées sous les dénominations SILLITIN N85 (3 µm), SILLITIN N87(3 µm), SILLITIN N82 (3 µm), SILLITIN V85 (4 µm) et SILLITIN V88 (4 µm) par la société HOFFMANN MINERAL, ou SUNSIL 130 (6-9 µm)par la société SUNJIN CHEMICAL, MSS-500-3 H (3 µm) par la société Kobo, SUNSPHERE H 51 (5 µm) par la société AGC SI-TECH, ainsi que les particules creuses de silice amorphe de forme ellipsoïdale commercialisées par Kobo sous la référence Silica Shells.

Les particules susceptibles d'être utilisées dans le cadre de l'invention peuvent être des particules de silicate insoluble.
On entend par "silicate insoluble", un silicate qui présente une solubilité dans l'eau inférieure à 0,5%, de préférence inférieure à 0,1% en poids à 25°C.
Les silicates peuvent être naturels ou chimiquement modifiés (ou synthétiques). Les silicates correspondent à de la silice éventuellement hydratée dont une partie des atomes de silicium sont remplacés par des cations métalliques comme Al3+, B3+, Fe3+, Ga3+, Be2+, Zn2+, Mg2+, Co3+, Ni3+, Na+, Li+, Ca2+, Cu2+.
Plus particulièrement, les silicates susceptible d'être utilisés dans le cadre de l'invention peuvent être choisis parmi (i) les argiles de la famille des smectites telles que les montmorillonites, les hectorites, les bentonites, les beidellites, les saponites, et (ii) les argiles de la famille des vermiculites, de la stévensite, des chlorites et les talcs ou la pyrophyllite. Ces argiles peuvent être d'origine naturelle ou synthétique. De préférence, on utilise les argiles qui sont cosmétiquement compatibles avec les matières kératiniques.
Le silicate peut être avantageusement choisi parmi la montmorillonite, la bentonite, l'hectorite, l'attapulgite, la sépiolite, les talcs et leurs mélanges.
On peut ainsi citer les composés commercialisés par la société LAPORTE sous la dénomination LAPONITE XLG et LAPONITE XLS.

Les particules de silicate sont avantageusement des particules de silicate de magnésium.
Les silicates de magnésium conformes à l'invention peuvent être d'origine naturelle ou synthétique. Le talc est particulièrement privilégié à titre de silicate de magnésium conforme à l'invention. Les talcs sont des silicates de magnésium hydratés comprenant le plus souvent en outre du silicate d'aluminium. La structure cristalline du talc consiste en des couches répétées d'un sandwich de brucite entre des couches de silice. Le talc conforme à l'invention peut plus particulièrement être choisi parmi ceux commercialisés sous les dénominations TALC SG-2000^{®} vendu par la société Nippon Talc, LUZENAC PHARMA M^{®} vendu par la société LUZENAC, J-68BC de US Corporation et MICRO ACE-P-3^{®} vendu par la société Nippon Talc.

Les silicates peuvent être modifiés avec un composé choisi parmi les amines quaternaires, les amines tertiaires, les acétates aminés, les imidazolines, les savons aminés, les sulfates gras, les alkyl aryl sulfonates, les oxides amines, et leurs mélanges.
Comme silicates susceptibles d'être utilisés, on ainsi peut citer les quaternium-18 bentonites telles que celles vendues sous les dénominations Bentone 3, Bentone 38, Bentone 38V par la société Rhéox; Tixogel VP par la société United Catalyst, Claytone 34, Claytone 40, Claytone XL par la société Southern Clay; les stéaralkonium bentonites telles que celles vendues sous les dénominations Bentone 27 par la société Rheox; Tixogel LG par la société United Catalyst; Claytone AF, Claytone APA par la société Southern Clay; les quaternium-18/benzalkonium bentonites telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel IPP, Bentone Gel ISD, Bentone Gel SS71, Bentone Gel VS8, Bentone Gel VS38 par la société Rhéox et Simagel M, Simagel SI 345 par la société Biophil.

Selon un mode de réalisation particulier, les silicates sont non modifiés.

De préférence le silicate insoluble de l'invention est un silicate de magnésium, et encore mieux un talc.

Les particules solides de silice ou de silicate insoluble peuvent être mises en oeuvre à une teneur allant de 0,01 à 5% en poids, de préférence de 0,02 à 1% en poids, et mieux de 0,04 à 0,8% en poids, par rapport au poids total de la composition.

### Milieu

La composition cosmétique selon l'invention se présente sous forme d'émulsion huile-dans-eau (phase hydroalcoolique), et peut comprendre en outre divers additifs. La composition est cosmétique, c'est-à-dire cosmétiquement acceptable, donc compatible avec les matières kératiniques telles que la peau du visage ou du corps, les cheveux, les cils, les sourcils et les ongles, et présentant généralement une couleur, une odeur et un toucher agréables, qui ne génèrent pas d'inconforts inacceptables (picotements, tiraillements, rougeurs), susceptibles de détourner le consommateur d'utiliser cette composition.

Ainsi, la composition selon l'invention peut comprendre un ou plusieurs parfums, par exemple en une quantité de 1 à 20% en poids, notamment de 2 à 15% en poids, par rapport au poids total de la composition (lorsqu'ils sont présents).
Les parfums sont des compositions contenant notamment les matières premières décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III. Il peut également s'agir de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

La composition selon l'invention peut en outre comprendre un ou plusieurs sels métalliques hydrosolubles.
Par "sel métallique", on entend selon la présente invention un sel d'un métal, c'est à dire d'un corps simple susceptible de libérer des cations simples (Dictionnaire de la Chimie et de ses Applications, DUVAL & DUVAL, 3ème édition, 1978, Technique et Documentation).
Par "sel métallique hydrosoluble", on entend tout sel métallique, cosmétiquement acceptable, susceptible d'être complètement dissous à l'état moléculaire dans une phase aqueuse liquide comprenant de l'eau ou un mélange d'eau/monoalcool en C₁-C₄.
Les sels métalliques hydrosolubles sont de préférence choisis parmi les sels hydrosolubles de métaux alcalins ou de métaux alcalino-terreux, et tout particulièrement parmi les sels de sodium ou de potassium; ou les sels de magnésium ou de calcium. Ces sels peuvent être par exemple des carbonates, des bicarbonates, des sulfates, des chlorures, des nitrates, des acétates, des hydroxydes, ainsi que des sels d'α-hydroxyacides ou des sels d'acides de fruits (citrate, tartrate, lactate, malate), ou encore des sels d'acides aminés (aspartate, arginate, glucocholate, fumarate).
De préférence, les sels métalliques hydrosolubles sont choisis parmi le chlorure de calcium et le chlorure de sodium; préférentiellement, il s'agit du chlorure de calcium.
Les sels métalliques hydrosolubles peuvent être présents en une quantité de 0,1 à 5% en poids, de préférence de 0,2 à 2% en poids, par rapport au poids total de la composition (lorsqu'ils sont présents).

La composition cosmétique peut en outre avantageusement comprendre au moins un constituant additionnel, usuel en cosmétique, tel que notamment des épaississants; des gélifiants; des tensioactifs; des agents de conditionnement; des silicones; des agents antichute; des agents antipelliculaires; des vitamines; des cires, des filtres solaires, des pigments; des agents nacrants et opacifiants, des agents séquestrants, des agents plastifiants, des conservateurs. Bien entendu, l'homme du métier veillera à choisir ce ou ces éventuels composés complémentaires et/ou leur quantité de manière telle que les propriétés avantageuses de la composition selon l'invention ne soient pas, ou substantiellement pas, altérées par l'adjonction envisagée.

La composition selon l'invention peut avantageusement se présenter sous forme d'une composition capillaire, à rincer ou non, notamment pour le lavage, le soin et/ou le conditionnement des cheveux; tout particulièrement, sous forme de composition capillaire de soin ou de conditionnement, à rincer ou non.

La composition selon l'invention peut être fabriquée selon les procédés connus des émulsions Pickering.

La composition selon l'invention peut se présenter sous la forme de spray ou d'aérosol, ou bien être conditionnée dans un flacon. La composition de l'invention peut être diffusée selon différents systèmes bien connus de l'homme de l'art, tel que des pulvérisateurs avec ou sans gaz sous pression. La composition selon l'invention peut également être appliquée sous forme de fines particules au moyen de dispositifs de pressurisation. Les dispositifs conformes à l'invention sont bien connus de l'homme de l'art et comprennent les pompes non-aérosols ou "atomiseurs", les dispositifs piézoélectriques, les récipients aérosols comprenant un propulseur ainsi que les pompes aérosols utilisant l'air comprimé comme propulseur. Ces derniers sont notamment décrits dans les brevets US 4,077,441 et US 4,850,517. Les compositions conditionnées en aérosol conformes à l'invention contiennent en général des agents propulseurs conventionnels tels que par exemple le diméthyléther, l'isobutane, le n-butane, le propane ou le trichlorofluorométhane.
La composition selon l'invention est préférentiellement biphasique et se présente préférentiellement sous forme de perles dans un milieu continu.
De préférence, la composition biphasique selon l'invention peut être agitée avant usage de manière à lui conférer une parfaite homogénéité dans l'ensemble de la composition.

La présente invention concerne également un procédé de traitement cosmétique, notamment capillaire, et tout particulièrement de conditionnement, des matières kératiniques, notamment des cheveux, qui consiste à appliquer une composition telle que décrite ci-dessus, de préférence après agitation, sur lesdites matières kératiniques, à effectuer optionnellement un rinçage par exemple avec de l'eau après un éventuel temps de pose. De préférence, on effectue un rinçage après un éventuel temps de pose.

La présente invention est illustrée plus en détails dans les exemples qui suivent.

### Exemple 1

On prépare une composition capillaire, pour le conditionnement des cheveux, comprenant (% en poids du produit commercial) :

| Composition | % en poids |
|---|---|
| Chlorure de calcium, 2 H₂O | 1 |
| Ethanol | 50 |
| Talc (taille : 7 microns) LUZENAC PHARMA M de Luzenac | 0,1 |
| Polyisobutène hydrogéné | 39,9 |
| Huile de tournesol (Huile de tournesol raffinée de la société Huileries de Lapalisse) | 1 |
| Parfum | 6 |
| Eau | Qsp 100% |

On obtient une composition sous forme d'émulsion huile-dans-eau contenant des perles en suspension dans un liquide parfaitement limpide et incolore. La taille des perles est de 1 à 5 mm.
Après un secouage manuel modéré de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes. Après 2 mois, l'apparence et les qualités organoleptiques du produit sont inchangées, aussi bien à température ambiante (25°C) qu'à 37°C.

### Exemple 2

On prépare une composition capillaire, pour le conditionnement des cheveux, comprenant (% en poids de produit commercial) :

| Composition | % en poids |
|---|---|
| Chlorure de calcium, 2 H₂O | 1,3 |
| Ethanol | 42 |
| Talc (taille : 5 microns) MICRO ACE P3 de Nippon Talc | 0,05 |
| Polyisobutène hydrogéné | 46 |
| Huile d'olive (huile d'olive vierge extra extraite à froid de la société Huileries de Lapalisse) | 5 |
| Parfum | 4 |
| D&C Yellow 5 + D&C Yellow 6 (hydrosoluble) | 0,00035 |
| Eau | Qsp 100% |

On obtient une composition sous forme d'émulsion huile-dans-eau (biphasique) contenant des perles en suspension dans un liquide parfaitement limpide de couleur jaune. La taille des perles est de 1 à 5 mm.
Après un secouage manuel modéré de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes.
Après 2 mois, l'apparence et les qualités organoleptiques du produit sont inchangées, aussi bien à 25°C qu'à 37°C.

### Exemple 3

On prépare une composition capillaire, pour le conditionnement des cheveux, comprenant (% en poids) :

| Composition | % en poids |
|---|---|
| Chlorure de calcium, 2 H₂O | 0,5 |
| Ethanol | 67 |
| Talc (taille : 2 microns) TALC 2755 USP BC de Brenntag | 0,2 |
| Polyisobutène hydrogéné | 25 |
| Huile de camélia (CAMELLIA OIL de la société Yokoseki) | 2 |
| Parfum | 3 |
| F&DC Blue 1 (hydrosoluble) | 0.0001 |
| Eau | Qsp 100% |

On obtient une composition sous forme d'émulsion huile-dans-eau (biphasique) contenant des perles en suspension dans un liquide parfaitement limpide de couleur bleue. La taille des perles est de 1 à 5 mm.
Après un secouage manuel modéré de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes.
Après 2 mois, l'apparence et les qualités organoleptiques du produit sont inchangées, aussi bien à 25°C qu'à 37°C.

### Exemple 4

On prépare une composition capillaire, pour le conditionnement des cheveux, comprenant (% en poids) :

| Composition | % en poids |
|---|---|
| Chlorure de calcium, 2 H₂O | 0.8 |
| Ethanol | 55 |
| Talc (MICRO ACE P3 de la société Nippon Talc) | 0.15 |
| Polyisobutène hydrogéné | 35 |
| Huile d'argan (ARGANIA SPINOSA OIL) * | 1 |
| Parfum | 6 |
| D&C Violet 2 (liposoluble) | 0.001 |
| Eau | Qsp 100% |

| | |
|---|---|
| * LIPOFRUCTYL ARGAN BE LS 9779 de la société Laboratoires Sérobiologiques (BASF) | |

On obtient une composition sous forme d'émulsion huile-dans-eau contenant des perles colorées (violet) en suspension dans un liquide parfaitement limpide et incolore. La taille des perles est de 1 à 5 mm.
Après agitation manuelle modérée de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes.
Après 2 mois, l'apparence et les qualités organoleptiques du produit sont inchangées, aussi bien à 25°C qu'à 37°C.

### Exemple 5

On prépare une composition capillaire, pour le conditionnement des cheveux, comprenant (% en poids) :

| Composition | % en poids |
|---|---|
| Chlorure de calcium, 2 H₂O | 1,3 |
| Ethanol | 42 |
| Talc (MICRO ACE P3 de la société Nippon Talc) | 0,05 |
| Polyisobutène hydrogéné | 46 |
| Huile d'avocat * | 5 |
| parfum | 4 |
| D&C Red 33 (hydrosoluble) | 0,0002 |
| Eau | Qsp 100% |

| | |
|---|---|
| * AVOCADO OIL RBD STAB RE3 de la société Jan Dekker | |

On obtient une composition sous forme d'émulsion huile-dans-eau contenant des perles en suspension dans un liquide parfaitement limpide et coloré (rouge). La taille des perles est de 1 à 5 mm.
Après agitation manuelle modérée de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes.
Après 2 mois, l'apparence et les qualités organoleptiques du produit sont inchangées, aussi bien à 25°C qu'à 37°C.

### Exemple 6

On prépare une composition capillaire, pour le conditionnement des cheveux, comprenant (% en poids) :

| Composition | % en poids |
|---|---|
| Chlorure de calcium, 2 H₂O | 0,46 |
| Ethanol | 67 |
| Talc (MICRO ACE P3 de la société Nippon Talc) | 0,2 |
| Polyisobutène hydrogéné | 25 |
| Huile de camellia (CAMELLIA OLEIFERA SEED OIL) de la société YOKOSEKI | 2 |
| parfum | 3 |
| FD&C Yellow 6 (hydrosoluble) | 0.00035 |
| Eau | Qsp 100% |

On obtient une composition sous forme d'émulsion huile-dans-eau contenant des perles en suspension dans un liquide parfaitement limpide et coloré (jaune). La taille des perles est de 1 à 5 mm.
Après agitation manuelle modérée de 5 secondes, la composition s'homogénéise facilement et peut être vaporisée de façon uniforme par exemple à l'aide d'un flacon pompe standard. La composition apporte de la cosméticité et de la brillance aux cheveux, et parfume la chevelure.
Une fois le flacon reposé, la répartition originelle en biphase de la composition se fait au bout de 5 minutes.
Après 2 mois, l'apparence et les qualités organoleptiques du produit sont inchangées, aussi bien à 25°C qu'à 37°C.

## Revendications

1. Composition cosmétique sous forme d'émulsion huile-dans-eau, comprenant :
- une phase hydroalcoolique continue comprenant de l'eau et au moins un monoalcool en C₁-C₄; l'eau étant présente dans la composition à une teneur allant de 0,5 à 10% en poids par rapport au poids total de la composition;
- de 0,1 à 10% en poids, par rapport au poids total de la composition, d'une ou plusieurs huiles végétales de type triglycérides; et
- des particules choisies parmi les particules de silicate insoluble, les particules de silice, et leurs mélanges; lesdites particules présentant une taille inférieure ou égale à 10 µm.

2. Composition cosmétique selon la revendication 1, dans laquelle la ou les huiles végétales de type triglycérides sont présentes à raison de 0,2 à 8% en poids, notamment de 0,5 à 6% en poids.

3. Composition cosmétique selon l'une des revendications précédentes, dans laquelle les huiles végétales de type triglycérides sont choisies parmi l'huile d'olive; l'huile d'avocat; l'huile d'argan; l'huile de camélia, l'huile de palme, l'huile de germe de mais, l'huile de graine de coton, l'huile d'arachide, l'huile de graine de citrouille, l'huile de soja, l'huile de sésame, l'huile de germe de blé, l'huile de babassu, l'huile de coco, l'huile de colza, l'huile d'amande, l'huile de lin, l'huile de carthame, l'huile de graine de tournesol, ainsi que leurs mélanges.

4. Composition cosmétique selon l'une des revendications précédentes, dans laquelle les huiles végétales de type triglycérides sont choisies parmi les huiles végétales de type triglycérides dont la teneur en acide gras en C16:0 est supérieure ou égale à 7,5%.

5. Composition cosmétique selon l'une des revendications précédentes, comprenant en outre une ou plusieurs huiles additionnelles choisies parmi les huiles hydrocarbonées apolaires, les alcools gras liquides et les esters liquides autres que les triglycérides.

6. Composition cosmétique selon la revendication 5, dans laquelle l'huile additionnelle est choisie parmi les isoparaffines hydrogénées ou non, l'alcool oléique, l'alcool isostéarylique, l'octyldodécanol et le myristate d'isopropyle.

7. Composition cosmétique selon l'une des revendications précédentes, dans laquelle le monoalcool est l'éthanol.

8. Composition cosmétique selon l'une des revendications précédentes, dans laquelle le ou les monoalcools sont présents dans la composition à une teneur comprise entre 20 et 80% en poids, de préférence entre 40 et 70% en poids, par rapport au poids total de la composition.

9. Composition cosmétique selon l'une des revendications précédentes, dans laquelle l'eau est présente dans la composition à une teneur allant de 1 à 5% en poids par rapport au poids total de la composition.

10. Composition cosmétique selon l'une des revendications précédentes, dans laquelle les particules présentent une taille comprise entre 1 et 10 µm, préférentiellement entre 1,5 et 8 µm.

11. Composition cosmétique selon l'une des revendications précédentes, dans laquelle les particules sont des particules de silicate de magnésium, de préférence de talc.

12. Composition cosmétique selon l'une des revendications précédentes, dans laquelle les particules de silice et/ou de silicate insoluble sont présentes dans la composition à une teneur comprise entre 0,01 et 5% en poids, de préférence entre 0,02 et 1% en poids, mieux de 0,04 à 0,8% en poids, par rapport au poids total de la composition.

13. Composition cosmétique selon l'une des revendications précédentes, comprenant en outre un ou plusieurs parfums, notamment en une quantité de 1 à 20% en poids, notamment de 2 à 15% en poids, par rapport au poids total de la composition

14. Composition cosmétique selon l'une des revendications précédentes, comprenant en outre un ou plusieurs sels métalliques hydrosolubles; de préférence choisis parmi le chlorure de calcium et le chlorure de sodium.

15. Composition cosmétique selon l'une des revendications précédentes, se présentant sous forme biphasique, préférentiellement sous forme de perles dans un milieu continu.

16. Composition cosmétique selon l'une des revendications précédentes, se présentant sous forme d'une composition capillaire, à rincer ou non, notamment pour le lavage, le soin et/ou le conditionnement des cheveux; tout particulièrement, sous forme de composition capillaire de soin ou de conditionnement, à rincer ou non.

17. Procédé de traitement cosmétique, notamment capillaire, et tout particulièrement de conditionnement, des matières kératiniques, notamment des cheveux, qui consiste à appliquer une composition selon l'une des revendications 1 à 16, de préférence après agitation, sur lesdites matières kératiniques, à effectuer optionnellement un rinçage par exemple avec de l'eau après un éventuel temps de pose.

## Patentansprüche

1. Kosmetische Zusammensetzung in Form einer Öl-in-Wasser-Emulsion, umfassend:
- eine kontinuierliche wässrig-alkoholische Phase, die Wasser und mindestens einen C₁-C₄-Monoalkohol umfasst; wobei das Wasser in der Zusammensetzung in einem Gehalt im Bereich von 0,5 bis 10 Gew.-%, bezogen auf das Gesamtgewicht der Zusammensetzung, vorliegt;
- 0,1 bis 10 Gew.-%, bezogen auf das Gesamtgewicht der Zusammensetzung, eines oder mehrerer pflanzlicher Öle vom Triglycerid-Typ und
- Teilchen, die aus Teilchen von unlöslichem Silikat, Teilchen von Siliciumdioxid und Mischungen davon ausgewählt sind; wobei die Teilchen eine Größe kleiner gleich 10 µm aufweisen.

2. Kosmetische Zusammensetzung nach Anspruch 1, wobei das pflanzliche Öl bzw. die pflanzlichen Öle vom Triglycerid-Typ in einer Menge von 0,2 bis 8 Gew.-%, insbesondere von 0,5 bis 6 Gew.-%, vorliegen.

3. Kosmetische Zusammensetzung nach einem der vorhergehenden Ansprüche, wobei die pflanzlichen Öle vom Triglycerid-Typ aus Olivenöl; Avocadoöl; Arganöl; Kamelienöl, Palmöl, Maiskeimöl, Baumwollsaatöl, Erdnussöl, Kürbiskernöl, Sojaöl, Sesamöl, Weizenkeimöl, Babassuöl, Kokosöl, Colzaöl, Mandelöl, Leinöl, Safloröl, Sonnenblumenkernöl sowie Mischungen davon ausgewählt sind.

4. Kosmetische Zusammensetzung nach einem der vorhergehenden Ansprüche, wobei die pflanzlichen Öle vom Triglycerid-Typ aus pflanzlichen Ölen vom Triglycerid-Typ mit einem Gehalt an C16:0-Fettsäure größer gleich 7,5% ausgewählt sind.

5. Kosmetische Zusammensetzung nach einem der vorhergehenden Ansprüche, die außerdem ein oder mehrere zusätzliche Öle, die aus apolaren Kohlenwasserstoffölen, flüssigen Fettalkoholen und flüssigen Estern, die von Triglyceriden verschieden sind, ausgewählt sind, umfasst.

6. Kosmetische Zusammensetzung nach Anspruch 5, wobei das zusätzliche Öl aus gegebenenfalls hydrierten Isoparaffinen, Oleylalkohol, Isostearylalkohol, Octyldodecanol und Isopropylmyristat ausgewählt ist.

7. Kosmetische Zusammensetzung nach einem der vorhergehenden Ansprüche, wobei es sich bei dem Monoalkohol um Ethanol handelt.

8. Kosmetische Zusammensetzung nach einem der vorhergehenden Ansprüche, wobei der Monoalkohol bzw. die Monoalkohole in der Zusammensetzung in einem Gehalt zwischen 20 und 80 Gew.-%, vorzugsweise zwischen 40 und 70 Gew.-%, bezogen auf das Gesamtgewicht der Zusammensetzung, vorliegt bzw. vorliegen.

9. Kosmetische Zusammensetzung nach einem der vorhergehenden Ansprüche, wobei das Wasser in der Zusammensetzung in einem Gehalt im Bereich von 1 bis 5 Gew.-%, bezogen auf das Gesamtgewicht der Zusammensetzung, vorliegt.

10. Kosmetische Zusammensetzung nach einem der vorhergehenden Ansprüche, wobei die Teilchen eine Größe zwischen 1 und 10 µm, bevorzugt zwischen 1,5 und 8 µm, aufweisen.

11. Kosmetische Zusammensetzung nach einem der vorhergehenden Ansprüche, wobei es sich bei den Teilchen um Teilchen von Magnesiumsilicat, vorzugsweise von Talk, handelt.

12. Kosmetische Zusammensetzung nach einem der vorhergehenden Ansprüche, wobei die Teilchen von Siliciumdioxid und/oder von unlöslichem Silikat in der Zusammensetzung in einem Gehalt zwischen 0,01 und 5 Gew.-%, vorzugsweise zwischen 0,02 und 1 Gew.-%, noch besser von 0,04 bis 0,8 Gew.-%, bezogen auf das Gesamtgewicht der Zusammensetzung, vorliegen.

13. Kosmetische Zusammensetzung nach einem der vorhergehenden Ansprüche, die außerdem einen oder mehrere Duftstoffe, insbesondere in einer Menge von 1 bis 20 Gew.-%, insbesondere von 2 bis 15 Gew.-%, bezogen auf das Gesamtgewicht der Zusammensetzung, umfasst.

14. Kosmetische Zusammensetzung nach einem der vorhergehenden Ansprüche, die außerdem ein oder mehrere wasserlösliche Metallsalze, die vorzugsweise aus Calciumchlorid und Natriumchlorid ausgewählt sind, umfasst.

15. Kosmetische Zusammensetzung nach einem der vorhergehenden Ansprüche, die in zweiphasiger Form vorliegt, vorzugsweise in Form von Perlen in einem kontinuierlichen Medium.

16. Kosmetische Zusammensetzung nach einem der vorhergehenden Ansprüche, die in Form einer Wash-out- oder Leave-in-Haarzusammensetzung vorliegt, insbesondere zur Wäsche, Pflege und/oder Konditionierung der Haare, ganz besonders in Form einer Wash-out- oder Leave-in-Haarzusammensetzung zur Pflege oder Konditionierung.

17. Verfahren zur kosmetischen Behandlung, insbesondere Haarbehandlung und ganz besonders Konditionierungsbehandlung, von Keratinmaterialien, insbesondere dem Haar, das darin besteht, dass man auf die Keratinmaterialien eine Zusammensetzung nach einem der Ansprüche 1 bis 16 aufbringt, vorzugsweise nach Schütteln, und nach einer fakultativen Einwirkungszeit gegebenenfalls eine Spülung, beispielsweise mit Wasser, vornimmt.

## Claims

1. Cosmetic composition in the form of an oil-inwater emulsion, comprising:
- a continuous aqueous-alcoholic phase comprising water and at least one C₁-C₄ monoalcohol, the water being present in the composition at a content ranging from 0.5% to 10% by weight relative to the total weight of the composition; and
- from 0.1% to 10% by weight, relative to the total weight of the composition, of one or more vegetable oils of triglyceride type; and
- particles chosen from particles of insoluble silicate, particles of silica, and mixtures thereof, said particles having a size of less than or equal to 10 µm.

2. Cosmetic composition according to Claim 1, in which the vegetable oil(s) of triglyceride type is (are) present in a proportion of from 0.2% to 8% by weight, in particular from 0.5% to 6% by weight.

3. Cosmetic composition according to either of the preceding claims, in which the vegetable oils of triglyceride type are chosen from olive oil; avocado oil; argan oil; camellia oil, palm oil, maize germ oil, cottonseed oil, peanut oil, pumpkin seed oil, soya bean oil, sesame oil, wheatgerm oil, babassu oil, coconut oil, rapeseed oil, almond oil, linseed oil, safflower oil, sunflower seed oil, and also mixtures thereof.

4. Cosmetic composition according to one of the preceding claims, in which the vegetable oils of triglyceride type are chosen from vegetable oils of triglyceride type of which the C16:0 fatty acid content is greater than or equal to 7.5%.

5. Cosmetic composition according to one of the preceding claims, also comprising one or more additional oils chosen from non-polar hydrocarbon-based oils, liquid fatty alcohols and liquid esters other than the triglycerides.

6. Cosmetic composition according to Claim 5, in which the additional oil is chosen from hydrogenated or non-hydrogenated isoparaffins, oleyl alcohol, isostearyl alcohol, octyldodecanol and isopropyl myristate.

7. Cosmetic composition according to one of the preceding claims, in which the monoalcohol is ethanol.

8. Cosmetic composition according to one of the preceding claims, in which the monoalcohol(s) is (are) present in the composition at a content of between 20% and 80% by weight, preferably between 40% and 70% by weight, relative to the total weight of the composition.

9. Cosmetic composition according to one of the preceding claims, in which the water is present in the composition at a content ranging from 1% to 5% by weight relative to the total weight of the composition.

10. Cosmetic composition according to one of the preceding claims, in which the particles have a size of between 1 and 10 µm, preferentially between 1.5 and 8 µm.

11. Cosmetic composition according to one of the preceding claims, in which the particles are particles of magnesium silicate, preferably of talc.

12. Cosmetic composition according to one of the preceding claims, in which the particles of silica and/or of insoluble silicate are present in the composition at a content of between 0.01% and 5% by weight, preferably between 0.02% and 1% by weight, better still from 0.04% to 0.8% by weight, relative to the total weight of the composition.

13. Cosmetic composition according to one of the preceding claims, also comprising one or more fragrances, in particular in an amount of from 1% to 20% by weight, in particular from 2% to 15% by weight, relative to the total weight of the composition.

14. Cosmetic composition according to one of the preceding claims, also comprising one or more water-soluble metal salts, preferably chosen from calcium chloride and sodium chloride.

15. Cosmetic composition according to one of the preceding claims, which is in two-phase form, preferentially in the form of beads in a continuous medium.

16. Cosmetic composition according to one of the preceding claims, which is in the form of a rinse-off or leave-on hair composition, in particular for washing, caring for and/or conditioning the hair, most particularly in the form of a rinse-off or leave-on care or conditioning hair composition.

17. Process for cosmetic treatment, in particular cosmetic hair treatment, and most particularly for conditioning, of keratin materials, in particular the hair, which consists in applying a composition according to one of Claims 1 to 16, preferably after shaking, to said keratin materials, and in optionally rinsing for example with water after an optional leave-on time.
